**Europäisches Patentamt**

**European Patent Office**

**Office européen des brevets**

(19)

(11) Publication number : **0 378 519 B1**

# EUROPEAN PATENT SPECIFICATION

(12)

(45) Date of publication of patent specification :
**01.06.94 Bulletin 94/22**

(51) Int. Cl.⁵ : **A61K 7/032,** A61K 7/043,
A61K 7/06, A61K 31/725,
A61K 31/73

(21) Application number : **90830002.3**

(22) Date of filing : **02.01.90**

(54) Compound for protecting, nourishing and treating hair, scalps eyelashes and nails.

(30) Priority : **10.01.89 IT 1904089**

(43) Date of publication of application :
**18.07.90 Bulletin 90/29**

(45) Publication of the grant of the patent :
**01.06.94 Bulletin 94/22**

(84) Designated Contracting States :
**BE CH DE ES FR GB GR LI NL**

(56) References cited :
**EP-A- 0 066 283**
**FR-A- 2 094 109**
**FR-A- 2 591 889**
**US-A- 4 767 463**

(73) Proprietor : **PROMO INTERNATIONAL S.r.l.**
**Via Melloni, 8**
**I-20129 Milano (IT)**

(72) Inventor : **Imperatori, Diana, Promo**
**International S.r.l.**
**Via Melloni, 8**
**I-20129 Milano (IT)**

(74) Representative : **Cicogna, Franco**
**Ufficio Internazionale Brevetti**
**Dott.Prof. Franco Cicogna**
**Via Visconti di Modrone, 14/A**
**I-20122 Milano (IT)**

## Description

The present invention relates to a compound which can be used as a base element for preparing several cosmetic and curative products for hair,scalp,eyelashes and nails.

Several compounds are already known which comprise,in their formulation,one or more substances which are considered as useful for protecting, nourishing and treating hair,scalp,as well as eyelashes and nails.

These substances,which have a more or less true efficacy,are derived from the chemical field, vegetable field or animal field.

FR-A-2094109 discloses the use of glucosaminoglicans in a composition for treating hair, the glucosaminoglicans are present in this prior compound in an amount of 0.1 to 15% and preferably in an amount of typically 2%.

US-A-4767463 relates to a composition of glucosaminoglicans with other polysaccharides of vegetal origin combined by means of a chemical reaction with cationic substances, generally of a polymeric nature.

FR-A-2591889 relates exclusively to the use of glucosaminoglicans in shampoos and the like.

EP-A-0066283 relates to the use of glucosaminoglicans, which are combined with several substances such as metal cations, ascorbic acid and the like.

In this connection a great importance has been addressed to the study of the activity of glucose-aminoglicans which comprise polysaccharides of animal nature which are mainly present in the main substance or "matrix" which forms the medium in which the cells are embedded and in which the nourishing exchanges between the cells and hematic system occur.

The above mentioned glucosaminoglicans,as main components of the basic substance,are essential for this exchanges which are very important for the life of the cells.

Thus,it should be obvious that these substances,comprising polysaccharides,would be able of providing a remarkable protecting,nourishing and regenerative action in particular on hair,scalp,eyelashes and nails.

In fact,it has been found that glucosaminoglicans,which have been tested both in topical applications and in intradermatic injections,provide on the animal pile system and human hair a self-evident trophic action.

More specifically,in the case of the animal pile they stimulate the growth of the pile coat,which becomes stronger and with a deeper color;in the case of the human beings,on the other hand,these substances reduce the hair loss,scurf and seborrhea,and aid a growth of stronger hair.

The strict relationship of glucosaminoglicans,pile and hair is demonstrated by the fact that the first increase during the growth period,while decreases during the rest period.

Since an increased concentration of these natural polymers also induce a better circulation of the blood,at the pile follicle level,it has been considered as rational to use these substances as trophic elements for combatting alopecia caused by wants,lacks and alterations of the metabolism,at the pile follicle level.

However,studies tending to extract and purify the individual glucosaminoglicans have shown that they,as individually taken,preserve the tricogenetic activity,but the less purified mixture is proportionately more active,both with respect to the single pure components and to a mixture thereof.

From the above,it can be deduced that the other substances (proteins,polypeptides,glycopeptides,nucleoderivates and in general polyanions) included in the entire extract cooperate with the glucosaminoglicans,in a synergistic way,to improve the activity thereof.

## SUMMARY OF THE INVENTION

The main object of the present invention is to meet with the above mentioned requirements,by providing a compound,essentially based on glucosaminoglicans,associated with other polyanions and macromolecules,of differently depolymeried types,which compound can be used as a basic element for preparing several products for treating hair,scalp,eyelashes and nails.

Another object of the invention is to provide such a compound,which will be called " POLYANION" hereinafter,which is very efficient in promoting the exchange of nourishing elements,removal of the metabolic waste,local blood circulation,as well as hydratation,prevention of cell attack of exogenous and endogenous nature,and aiding the regeneration and homeostasis of the pile follicle.

More specifically,the assembly of the biologic macromolecules,which reproduce the natural environment tending to favour the hair growth and extend the duration of the pile growth period,that is the subject POLYANION,is defined by the following average composition:
- glucosaminoglicans        45%
- scleroproteins        15%
- nucleic acids        15%
- proteins and peptides        25%

This assembly,the composition of which can vary within limits of ± 20%,can provide a stronger and more complete tricogenesis with respect to that provided by the glucosaminoglicans taken individually:it would be apparent that the presence of other biologic macromolecules improves and aids the activity of the glusocaminoglicans at the pile follicle level.

As stated,the POLYANION formulation according to the present invention provides,on hair and scalp,a strong tricogenetic,trophic,protective,regenerating,anti-sebum,anti-pityriasis activity which,by normalizing the functions and cycles,is such as to greatly extend the life period of hair.

Thus,owing to its disclosed characteristics,this POLYANION can be advantageously be used in the formulation of several products for treating hair,scalp,eyelashes and nails.

Some examplary embodiments of the compound according to the present invention will be given in the following disclosure.

REGULAR HAIR LOTION

POLYANION  0.5 %
ALCOHOL     30 %
WATER         the balance to 100 ml

STRONG HAIR LOTION

POLYANION                                    2 %
ALCOHOL                                      20 %
PRESERVATIVES AND PERMFUME  as required
WATER                                          the balance to 100 ml

EYELASH GEL

POLYANION                                    1    %

CARBOXYVINYL RESIN                      0.6   %

NEUTRALIZING AND PRESERVATIVE

AGENTS  and PERFUME                     as required

WATER                                          the balance to 100 ml

NAIL REGENERATING CREAM

POLYANION                                    1.5 %
SELF-EMULSIFYING BASE                  10 %
VEGETAL OIL                                    8 %
PRESERVATIVES AND PERFUME         as required
WATER                                          the balance to 100 ml

HAIR PROTECTIVE SHAMPOO

POLYANION                                    0.5 %
ANIONIC CLEANSING AGENTS            8 %
NON IONIC CLEANSING AGENTS       3 %
AMPHOTERIC CLEANSING AGENTS    2 %
PRESERVATIVES AND PERFUME         as required
WATER                                          the balance to 100 ml

The subject POLYANION can also be used for preparing hair balsams or hair gum based products for treating and protecting hair,eyelashes nails and the pile system in general.

Moreover,in these formulations,with the POLYANION can be associated other substances for balancing and stimulating the pile system,such as: vitamins,aminoacids,vessel dilaters,vegetable extracts and the like.

The invention has been disclosed and illustrated with reference to preferred embodiments.

## Claims

1. A compound for protecting, nourishing and treating hair, scalp, eyelashes and nails, comprising elements controlling nourishing exchanges of cells and blood, and, in particular, glucosaminoglicans, characterized in that said glucosaminoglicans are in synergic mixture with scleroproteins, nucleic acids, proteins and peptides, and that in said mixture said glucosaminoglicans, scleroproteins, nucleic acids, proteins and peptides are present with the following average proportions:
   - glucosaminoglicans        45%
   - scleroproteins        15%
   - nucleic acids        15%
   - proteins and peptides        25%

2. A compound according to Claim 1, characterized in that each said average proportions of said glucosaminoglicans, scleroproteins, nucleic acids, proteins and peptides can vary within limits of ± 20%.

3. A product for treating hair, scalp, eyelashes and nails, characterized in that said product comprises a compound according to Claim 1.

## Patentansprüche

1. Eine Verbindung zum Schutz, zur Ernährung und zur Behandlung der Haare, der Kopfhaut, der Augenwimpern und Nägel, umfassend Grundstoffe, die den Nährstoffaustausch von Zellen und Blut kontrollieren, und, insbesondere Glucosaminoglycane, dadurch gekennzeichnet, daß diese Glucosaminoglycane in synergetischem Gemisch mit Skleroproteinen, Nucleinsäuren, Proteinen und Peptiden sind, und daß in diesem Gemisch diese Glucosaminoglycane, Skleroproteine, Nucleinsäuren, Proteine und Peptide in den folgenden Durchschnittsverhältnissen enthalten sind:
   - Glucosaminoglycane        45%
   - Skleroproteine        15%
   - Nucleinsäuren        15%
   - Proteine und Peptide        25%

2. Eine Verbindung gemäß Anspruch 1, dadurch gekennzeichnet, daß jedes dieser Durchschnittsverhältnisse dieser Glucosaminoglycane, Skleroproteine, Nucleinsäuren, Proteine und Peptide innerhalb der Grenzen von ± 20% variieren kann.

3. Ein Produkt zur Behandlung von Haar, Kopfhaut, Augenwimpern und Nägeln, dadurch gekennzeichnet, daß dieses Produkt ein Gemisch gemäß Anspruch 1 umfaßt.

## Revendications

1. Composé pour la protection, la nutrition et le traitement des cheveux, du cuir chevelu, des cils et des ongles comprenant des constituants qui contrôlent les échanges de nutrition des cellules et du sang et, en particulier, des glucosaminopolysaccharides, caractérisé en ce que lesdits glucosamino-polysaccharides forment une mixture sinérgique avec des scléroprotéines, des acides nucléiques, des protéines et des peptides, et que les proportions moyennes desdits glucosaminopolysaccharides, scléroprotéines, acides nucléiques, protéines et peptides dans ladite mixture sont les suivantes:
   - glucosaminopolysaccharides        45%
   - scléroprotéines        15%
   - acides nucléiques        15%
   - protéines et peptides        25%

2. Composé selon la Revendication 1 caractérisé en ce que chacune desdites proportions moyennes desdits glucosaminopolysaccharides, scléroprotéines, acides nucléiques, protéines et peptides peut varier dans un marge de ±20%.

3. Un produit pour le traitement des cheveux, du cuir chevelu, des cils et des ongles, caractérisé en ce que ledit produit comprend un composé selon la Revendication 1.